(19) Europäisches Patentamt

European Patent Office

Office européen des brevets

(11) Veröffentlichungsnummer: **0 535 073 B1**

(12) **EUROPÄISCHE PATENTSCHRIFT**

(45) Veröffentlichungstag der Patentschrift: **09.08.95**

(21) Anmeldenummer: **91911456.1**

(22) Anmeldetag: **06.06.91**

(86) Internationale Anmeldenummer:
**PCT/EP91/01050**

(87) Internationale Veröffentlichungsnummer:
**WO 91/19559 (26.12.91 91/29)**

(51) Int. Cl.⁶: **B01D 53/22**, B01J 10/00,
C07B 41/12

Die Akte enthält technische Angaben, die nach
dem Eingang der Anmeldung eingereicht wurden
und die nicht in dieser Patentschrift enthalten sind.

(54) **VERFAHREN ZUM DURCHFÜHREN EINER GLEICHGEWICHTSREAKTION UNTER ANWENDUNG VON DÄMPFEPERMEATION.**

(30) Priorität: **15.06.90 DE 4019170**

(43) Veröffentlichungstag der Anmeldung:
**07.04.93 Patentblatt 93/14**

(45) Bekanntmachung des Hinweises auf die
Patenterteilung:
**09.08.95 Patentblatt 95/32**

(84) Benannte Vertragsstaaten:
**DE ES FR IT**

(56) Entgegenhaltungen:
**EP-A- 9 385**
**DE-A- 3 134 539**
**DE-C- 924 689**
**US-A- 2 956 070**

(73) Patentinhaber: **Henkel Kommanditgesellschaft auf Aktien**

**D-40191 Düsseldorf (DE)**
(84) Benannte Vertragsstaaten:
**DE FR**

(73) Patentinhaber: **BLUM, Stephan**
**Volksgartenstrasse 23**
**D-40764 Langenfeld (DE)**
(84) Benannte Vertragsstaaten:
**ES IT**

(73) Patentinhaber: **GUTSCHE, Bernhard, Dr.**
**Kalstert 96**
**D-40724 Hilden (DE)**
(84) Benannte Vertragsstaaten:
**ES IT**

Chemistry Letters, N 10,1987, S.2053-2056;
Kita et al., "The esterification of oleic acid
with ethanol accompanied by membrane separation"

PATENT ABSTRACTS OF JAPAN, vol. 11, no.
307 (C-450)(2754) 7. Oktober 1987 & JP-A-62
96 453 (JAPAN ORGANO CO. LTD.) 2. Mai
1987

⑦₃ Patentinhaber: **JEROMIN, Lutz, Dr.**
**Am Bandsbusch 88**
**D-40723 Hilden (DE)**
⑧₄ Benannte Vertragsstaaten:
**ES IT**

⑦₃ Patentinhaber: **Yüksel, Levent, Dr.**
**Marbacher Strasse 13**
**D-40597 Düsseldorf (DE)**
⑧₄ Benannte Vertragsstaaten:
**ES IT**

⑦₂ Erfinder: **BLUM, Stephan**
**Volksgartenstrasse 23**
**D-4018 Langenfeld (DE)**
Erfinder: **GUTSCHE, Bernhard**
**Lessingstrasse 5 A**
**D-4010 Hilden (DE)**
Erfinder: **JEROMIN, Lutz**
**Am Bandsbusch 88**
**D-4010 Hilden (DE)**
Erfinder: **YÜKSEL, Levent**
**Marbacher Str. 13**
**D-4000 Düsseldorf 13 (DE)**

**Beschreibung**

Die Erfindung betrifft ein Verfahren zum Durchführen einer Gleichgewichtsreaktion, bei der eines oder mehrere Produkte mittels Dämpfepermeation abgetrennt werden. Dabei wird die Dampfphase des Reaktionsgemisches im Reaktor dem zur Durchführung der Dämpfepermeation vorgesehenen Membranmodul als Feed zugeleitet.

Membranverfahren zur Fraktionierung von Gemischen mit organischen Komponenten sind in der Literatur ausführlich dargestellt, z. B. Rautenbach u. a. in Chem. Ing. Techn. 61 (1989) S. 535 - 544. Zum Einsatz der Pervaporation und der Dämpfepermeation liegt ebenfalls schriftlicher Stand der Technik vor, z.B. DE-C-3610011, EP-A-0294827 und EP-A-0273267.

Generell wird bei diesen Verfahren das zu trennende Stoffgemisch (Feed) an einer Membran entlanggeführt, die für eine Komponente durchlässig ist (semipermeabel). Durch ein an der feedabgewandten Seite (Permeatseite) der Membran angelegtes Vakuum wird ein Potentialgefälle erzeugt, das die Ausschleusung der vorwiegend permeierenden Komponente aus dem Feed zur Folge hat. Der entreicherte Feed wird als Retentat oder Konzentrat bezeichnet. Die im wesentlichen rein gewonnene, mit Hilfe der Membran abgetrennte Komponente wird Permeat genannt. Das Trennverhalten der Membran ist stark temperaturabhängig, wird aber nach oben durch die thermische Beständigkeit begrenzt. So wird z. B. die Temperaturbeständigkeit der Standardmembran der Firma GFT bis maximal 130 °C angegeben (G.F. Tusel et al., ACS Symposium Series 281 (1985), Reverse Osmosis and Ultrafiltration, S. Sourirajan (Ed.) 467 - 478). Eine Langzeitstabilität oberhalb von 100 °C konnte aber bisher nicht festgestellt werden. Das optimale Trennverhalten der GFT-Standardmembran liegt basierend auf Betriebserfahrungen zwischen 80 °C und 100 °C.

Die in den Druckschriften DE-C-3610011 und EP-A-0294827 beschriebenen Verfahren zeichnen sich dadurch aus, daß der Feed in flüssiger Form, knapp unterhalb des Siedepunktes bei gegebenem Systemdruck zugeführt wird. Der Systemdruck muß dabei so gewählt werden, daß die Temperatur des zu trennenden Stoffgemisches an der Membran möglichst der optimalen Temperatur entspricht, keinesfalls aber oberhalb der Maximaltemperatur liegt, da sonst irreversible Membranschädigungen auftreten.

Des weiteren hat der Kontakt des Feeds mit hydrophilen Membranen (GFT-Standardmembranen) eine Quellung der aktiven Schicht zur Folge, die einerseits eine Erhöhung der Permeabilität, andererseits aber einen Selektivitätsverlust zur Folge hat. Bei hohen Wassergehalten und gleichzeitig hohen Temperaturen kann die Quellung zu einer Ablösung der aktiven Schicht vom Stützgewebe und somit zur Zerstörung der Membran führen. Ferner muß eine chemische Membranschädigung durch eine der Feedkomponenten ausgeschlossen sein.

Somit ist der Einsatz der Pervaporation bei Reaktionen starken Einschränkungen unterworfen.

In EP-A-0273267 wird das Verfahren der Dämpfepermeation beschrieben. Die Dämpfepermeation unterscheidet sich von der Pervaporation dadurch, daß das zu trennende Stoffgemisch verdampft wird und der Dampf an der Membran entlanggeführt wird. Auch hier muß der Systemdruck so gewählt werden, daß die Dampftemperatur und damit die Temperatur des zu trennenden Stoffgemisches der Optimaltemperatur der Membran entspricht. Auch bei der Dämpfepermeation darf die Siedetemperatur des Feeds die Maximaltemperatur der Membran nicht überschreiten.

Die Dämpfepermeation bietet gegenüber der Pervaporation den Vorteil, daß zum einen die Membranquellung nicht zu beobachten ist, zum anderen auch die Gefahr einer chemischen Membranschädigung weitgehend ausgeschlossen ist.

Die Druckschriften US-A-2956070 und EP-A-0210055 befassen sich mit der Wasserauskreisung bei Veresterungsreaktionen unter Zuhilfenahme der Pervaporation.

Bedingt durch die oben angeführten Gründe wird die Pervaporation bei den in EP-A-021055 erwähnten Versuchen bei mäßigen Reaktionstemperaturen (50 - 90 °C durchgeführt. Hier wird auf die geringe Säurebeständigkeit der Membranen hingewiesen (max. 15 mol % des Reaktionsgemisches), die einen hohen Alkoholüberschuß bedingt.

Ein Verfahren zum Einsatz der Dämpfepermeation bei einer Veresterungsreaktion von Ölsäure mit Ethanol zur Wasserauskreisung wird bei Kita et al., Chemistry Letters (1987), Nr. 10, S 2053 - 2056 beschrieben. Hier wurden die Versuche bei mäßigen Temperaturen (85 °C) durchgeführt. Bei der hier verwendeten Versuchsanordnung darf die Reaktionstemperatur ebenfalls aus den oben genannten Gründen die Maximaltemperatur der Membran nicht überschreiten.

Nachteilig an allen oben angeführten Verfahren ist, daß die Fettsäure und der Alkohol gemischt vorgelegt werden. Der Systemdruck muß dabei immer so eingestellt werden, daß die Siedetemperatur des Gemisches gleich der für die Reaktion notwendigen Temperatur ist. Diese Temperatur darf aber aus den oben genannten Gründen nicht über die Maximaltemperatur der Membran (im Falle GFT-Standardmembran 100 °C) hinausgehen. Die meisten Reaktionstemperaturen liegen jedoch deutlich oberhalb von 100 °C, so daß die Maximaltemperaturen der meisten Membranen weit überschritten werden. Somit kann nach dem oben genannten Verfahren die Wasserauskreisung bei Veresterungsreaktionen bestenfalls bei der Maximaltemperatur der verwendeten Membran betrieben werden, was jedoch um so längere Reaktionszeiten zur Folge hat, je höher die optimale Reaktionstemperatur über dieser Maximaltemperatur liegt.

Der vorliegenden Erfindung liegt die Aufgabe zugrunde, bei dem Verfahren der eingangs genannten Art durch eine besondere Form der Reaktionsführung bei Gleichgewichtsreaktionen, z.B. Veresterungsreaktionen, bei hohen Reaktionstemperaturen (> 120 °C) hohe Reaktionsgeschwindigkeiten zu ermöglichen. Die Reaktion soll dabei bei beliebigen Temperaturen ablaufen können. Ferner soll auf die Forderung nach einer überdurchschnittlichen chemischen Beständigkeit der Membran, selbst bei starken Lösungsmitteln im Feed, verzichtet werden können, so daß im Fall der Veresterungsreaktion die Säure in reiner Form vorgelegt werden kann. Die Aufgabe besteht außerdem darin, die Möglichkeit zu schaffen, gleichzeitig eine optimale Reaktionstemperatur und eine davon unterschiedliche optimale Membrantemperatur einstellen zu können.

Diese Aufgabe wird erfindungsgemäß dadurch gelöst, daß die Reaktion in einem Blasenreaktor durchgeführt und das leichtersiedende Edukt durch das auf Reaktionstemperatur gehaltene, flüssige und getrennt vorgelegte Edukt geleitet wird, wobei die Reaktionstemperatur und der Reaktordruck so eingestellt werden, daß bei Reaktionstemperatur das leichtersiedende Edukt und das abzutrennende Produkt dampfförmig vorliegen, daß die Dämpfepermeation außerhalb des Reaktors unter geeigneter Temperatur, die abhängig vom Membranmaterial ist, vorgenommen und das bei der Dämpfepermeation entstehende Retentat zurückgeleitet und gemeinsam mit dem leichtersiedenden Edukt in den Reaktor geleitet wird. Die zum Betreiben des Membranmoduls erforderliche Partialdruckdifferenz kann sowohl durch ein Anlegen eines Vakuums auf der Permeatseite als auch durch einen Druckbetrieb auf der Feedseite erreicht werden.

Das erfindungsgemäße Verfahren hat den Vorteil, daß erheblich weniger Verfahrensbeschränkungen als im Stand der Technik vorliegen. Die Reaktion wird lediglich dadurch festgelegt, daß mindestens das leichtersiedende Edukt und die zu entfernende Produktkomponente bei Reaktionstemperatur dampfförmig vorliegen müssen. Das kann allein durch Vorgabe des Systemdruckes immer gewährleistet werden. Die uneingeschränkte Reaktionsführung wird dadurch möglich, daß zum einen durch die Anwendung des Blasenreaktors beide Edukte getrennt vorgelegt werden können und somit bei beliebiger Reaktionstemperatur durch Vorgabe des Systemdruckes die Temperatur des anfallenden Reaktionsdampfgemisches frei eingestellt werden kann. Außerdem wird durch die Anwendung der Dämpfepermeation ein Flüssigphasenkontakt zwischen der Membran und einer membranschädigenden Komponente vermieden.

In einer bevorzugten Ausgestaltung wird das erfindungsgemäße Verfahren zum Durchführen einer Veresterungsreaktion eingesetzt, bei dem Wasserdampf mittels Dämpfepermeation abgetrennt wird. Ein typisches Beispiel ist die Veresterung einer Fettsäure mit einem leichtersiedenden Alkohol. Nie oben beschrieben, werden die Fettsäure und der Alkohol getrennt vorgelegt.

Zwei Effekte werden zur Produktauskreisung, im Beispiel Wasserauskreisung, genutzt. Zum einen geht das abzutrennende Reaktionsprodukt bedingt durch die hohe Temperatur des vorgelegten Eduktes, im Beispiel Säure, sofort nach der Bildung in die Dampfphase über, und dampft aus dem Reaktionsvolumen aus, zum zweiten wird das abzutrennende Produkt (Wasser), bedingt durch den zirkulierenden Retentatdampf (Alkohol/Wasser-Dampfgemisch) aus dem Reaktionsgemisch desorbiert, d. h. das abzutrennende Reaktionsprodukt wird somit zusätzlich durch das durchperlende Retentat herausgestrippt.

Durch diese Reaktionsführung ist gegenüber dem Stand der Technik zum einen eine weitergehende Reduktion der aufzuwendenden Energien und Stoffmengen, zum anderen eine Chargen- und eine Reaktionszeitverkürzung zu erreichen. So kann insbesondere eine Umsetzung stöchiometrischer Mengen der Edukte bei kurzen Reaktionszeiten erfolgen (Beispiel I, Tabelle 2).

Um sowohl eine optimale Reaktortemperatur und auch eine optimale Membrantemperatur zu erreichen, obwohl diese Temperaturen in der Regel voneinander abweichen, wird gemäß einer bevorzugten Ausgestaltung vorgeschlagen, daß man das Membranmodul und/oder seine vom Reaktor herführende Zuleitung entsprechend der gewünschten Membrantemperatur erwärmt bzw. kühlt.

In letzterem Fall ist es denkbar, den Dampf direkt durch Verdampfungskühlung (Einblasen von flüssigem Alkohol) zu kühlen, um eine Modulkühlung zu umgehen. Diese Maßnahme führt jedoch zu einer Partialdruckabnahme der bevorzugt permeierenden Komponente und damit zu einer Verringerung der Triebkraft.

Der Reaktor wird erfindungsgemäß als Blasenreaktor ausgeführt, in dem beim Beispiel Veresterung die Fettsäure auf die notwendige Reaktionstemperatur gebracht wird. Der Alkohol wird flüssig, nahe am Siedepunkt, in den Reaktor gepumpt.

Da die Reaktionstemperatur (deutlich) über der Siedetemperatur des Alkohols liegt, verdampft der Alkohol bei Eintritt in die heiße Säure schlagartig, perlt in Blasenform durch das Reaktionsvolumen und sorgt so für eine gute Durchmischung. Das durch die Reaktions entstehende Wasser entweicht mit dem nicht umgesetzten Alkohol als überhitztes Dampfgemisch in den Dampfraum. Das Dampfgemisch wird einem Membranmodul zugeleitet, das als Aufnahme für die Membran dient. Diesem dampfförmigen Alkohol/Wasser-Gemisch wird durch das an der Membranpermeatseite angelegte Vakuum das Wasser entzogen. Das aus nicht umgesetztem Alkohol und nicht abgetrenntem Wasser bestehende verbleibende Retentat wird ggf. kondensiert und dem Reaktionsgemisch wieder zugeführt.

Es wird also Insbesondere vorgeschlagen, daß das Retentat zusammen mit dem leichtersiedenden Edukt in flüssigem Zustand in den Reaktor geleitet wird.

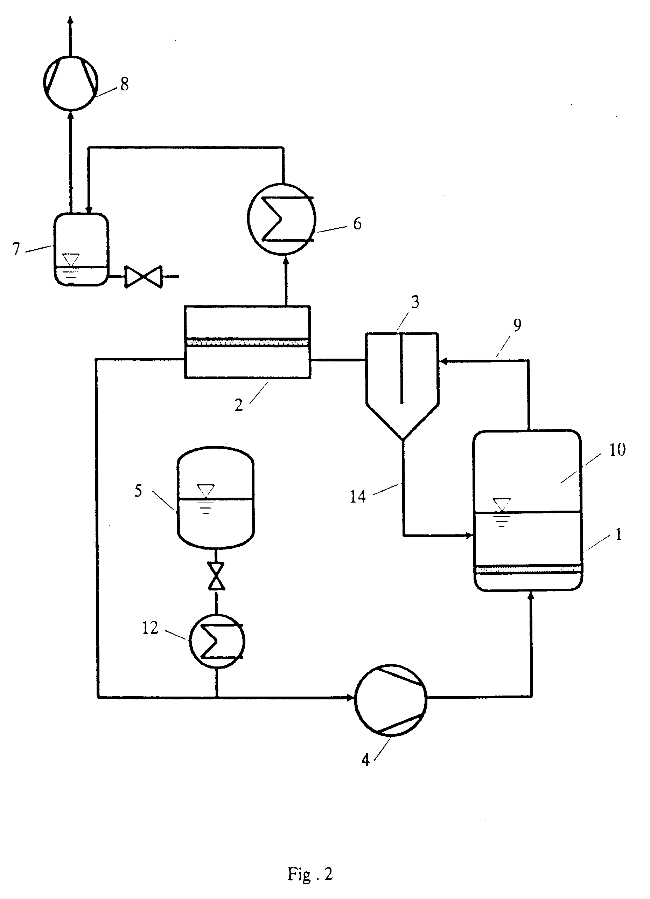

In einer anderen, ebenfalls vorteilhaften Ausgestaltung der Erfindung wird das Retentat zusammen mit dem leichtersiedenden Edukt in gasförmigem Zustand in den Reaktor geleitet (Fig. 2). Hier wird in Fall der Veresterung das Alkohol/Wasser-Gemisch gasförmig in den Reaktor eingeblasen. Der aus dem Reaktor austretende Dampfstrom wird analog zur oben beschriebenen Reaktionsführung durch das Membranmodul geführt und anschließend wieder angesaugt. Durch diese Umwälzung kann die energieaufwendige Verdampfung und somit auch die anschließende Kondensation des Retentats entfallen.

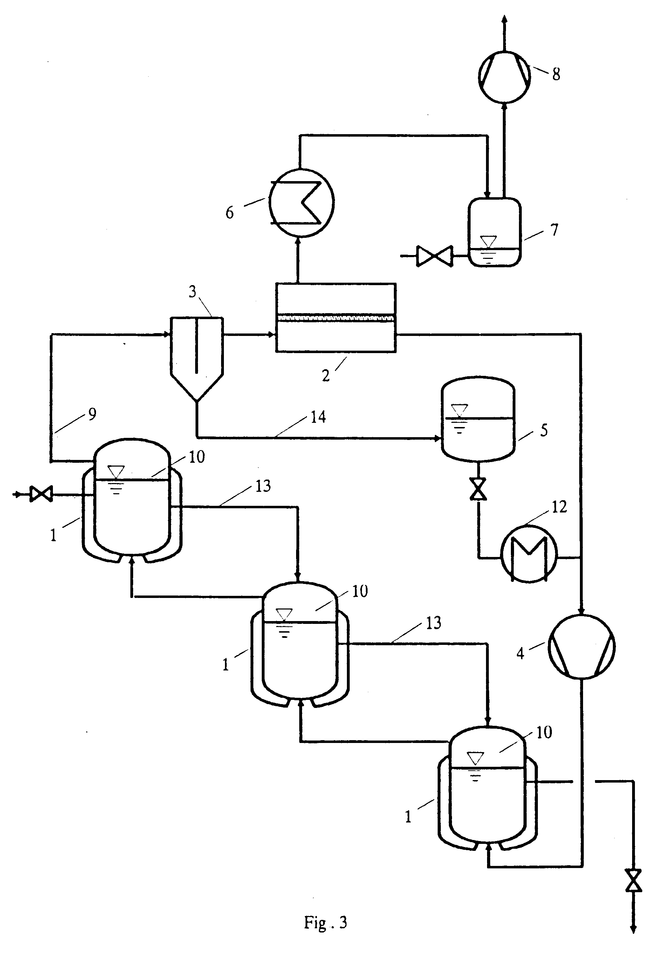

Besonders vorteilhaft für eine technische Realisierung ist es, wenn das höhersiedende flüssige und das leichtersiedende dampfförmige Edukt im Gegenstrom durch eine Rührkesselkaskade (Fig. 3) geleitet werden und die gasförmige Phase des Reaktionsgemisches erst nach Durchlaufen der Kaskade dem Membranmodul zugeleitet wird. Damit wird die benötigte Membranfläche gering gehalten. In einer anderen technischen Anwendung ist es vorteilhaft, wenn die Edukte durch eine Gegenstromkolonne geleitet werden und das entstehende gasförmige Reaktionsprodukt dem Membranmodul zugeleitet wird. Auch in diesem Fall ist nur eine geringe Membranfläche erforderlich.

Um eventuell im Dampf vorliegende geringe Mengen an membranschädlichem Edukt oder Produkt, z. B. Säure oder Ester im Veresterungsverfahren von der Membran fernzuhalten, ist es vorteilhaft, wenn den Brüden vor der Zuleitung zum Membranmodul eine Vorrichtung, z. B. ein Tropfenabscheider, zur Abscheidung mitgerissener Flüssigkeit vorgeschaltet ist.

Nachfolgend werden einige Ausführungsbeispiele anhand der Zeichnungen näher erläutert. Es zeigen

Figur 1 ein Fließdiagramm eines Veresterungsverfahrens gemäß der Erfindung, bei dem das Retentat in flüssigem Zustand in den Reaktor gepumpt wird,

Figur 2 ein Fließdiagramm entsprechend Figur 1, bei dem jedoch das Retentat in gasförmigem Zustand dem Reaktor zugeleitet wird,

Figur 3 eine Ausführung der Erfindung mit einer im Gegenstrom betriebenen Rührkesselkaskade.

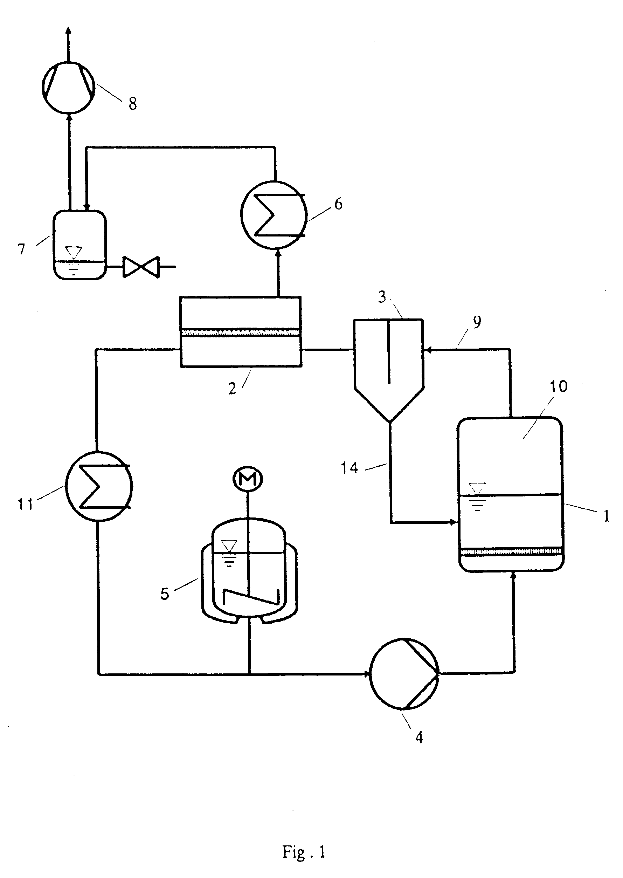

In dem erfindungsgemäßen Ausführungsbeispiel nach Figur 1 wird eine Veresterung durchgeführt. Der Alkohol wird aus einem separaten Vorlagebehälter 5 in flüssiger Form bei einer Temperatur nahe am Siedepunkt mit der Pumpe 4 in den Blasenreaktor 1 gepumpt. In diesem Reaktor wird Fettsäure auf der notwendigen Reaktionstemperatur gehalten. Beim Eintritt in die heiße Fettsäure verdampft der Alkohol schlagartig, perlt in Blasenform durch das Reaktionsgemisch und sorgt damit für eine gute Durchmischung. Das durch die Reaktion entstehende Wasser entweicht mit dem nicht umgesetzten Alkohol als überhitztes Dampfgemisch in den Dampfraum, die Dampfphase 10. Aus dem Dampfraum des Reaktors 1 führt eine Zuleitung 9 zu einem Membranmodul 2 über einen Tropfenabscheider 3. Das Membranmodul 2 dient als Träger für die Membran. Dem dampfförmigen, an der Membran entlangstreichenden Alkohol/Wasser-Gemisch wird das Wasser durch das an der Membranpermeatseite mit der Vakuumanlage 8 angelegte Vakuum entzogen. Das verbleibende Retentat besteht dann aus nicht umgesetztem Alkohol und nicht abgetrenntem Wasser und wird über einen geeigneten Kältemittelstrom im Kondensator 11 kondensiert und dem Reaktionsgemisch im Reaktor 1 wieder zugeführt. Das Permeat wird nach Kondensation an dem Permeatkondensator 6 im Permeatsammelbehälter 7 aufgefangen.

Das Ausführungsbeispiel nach Figur 2 entspricht dem nach Figur 1 mit dem Unterschied, daß das Alkohol/Wasser-Gemisch in gasförmigem Zustand mit dem Gasverdichter 4 in den Reaktor 1 eingeblasen wird. Der aus dem Reaktor 1 austretende Dampfstrom wird durch das Membranmodul 2 geführt und anschließend vom Gasverdichter 4 wieder angesaugt. Vorteilhaft an dieser Ausgestaltung ist die Einsparung der energieaufwendigen Verdampfung und damit auch der anschließenden Kondensation des Retentats.

In Figur 3 wird ein Fließdiagramm einer technischen Realisierung des erfindungsgemäßen Verfahrens dargestellt. Die Reaktion wird in mehreren Reaktoren 1 durchgeführt, die als Rührkesselkaskade im Gegenstrom zum zirkulierenden Dampfgemisch betrieben werden. Dabei ist sowohl eine kontinuierliche als auch eine diskontinuierliche Fahrweise möglich. Das aus dem letzten Reaktor austretende, mit dem bevorzugt permeierenden Stoff angereicherte Dampfgemisch wird im Membranmodul 2 entreichert und dem ersten Reaktor 1 wieder zugeführt. Ansonsten entspricht diese Ausführungsform der Ausführungsform nach Figur 2.

**Beispiel I**

Nachfolgend werden Ergebnisse einer entsprechend dem erfindungsgemäßen Verfahren durchgeführten Veresterungsreaktion von Myristinsäure mit Isopropanol (IPA) zu Isopropylmyristat (IPM) und Wasser angegeben.

Als Blasenreaktor diente ein Glasreaktor mit einem Reaktionsvolumen von 0,6 l (Ausgangsmenge $mol_o$ Myristinsäure = 2 mol). In einem aufgesetzten Modul wurde die GFT-Standardmembran mit einer auf das Reaktionsvolumen bezogenen Fläche von 115,5 $m^2/m^3$ IPM installiert. Bei dieser Membran handelt es sich um eine zweischichtige Kompositmembran mit einer aktiven Schicht aus vernetztem Polyvinylalkohol (PVA) und einer Stützschicht aus Polyacrylnitril. Der exakte Aufbau der Membran geht aus der Offenlegungsschrift DE-A-3220570 hervor. Bei Normaldruck wurde die Myristinsäure bei 120 °C vorgelegt, anschließend IPA flüssig bei ca. 82 °C in die heiße Säure gepumpt. Die eingesetzte IPA-Menge stand zur eingesetzten Myristinsäure im Molverhältnis 2,45 : 1.

Zunächst wurde die Reaktion ohne Wasserauskreisung betrieben, um die Wirkungsweise das Blasenreaktorkonzeptes zu quantifizieren. In Tab. 1 ist der Umsatz von Myristinsäure in Abhängigkeit von der Zeit dargestellt. Zum Vergleich ist der Gleichgewichtsumsatz eines Batchreaktors angegeben. Der deutlich bessere Umsatz im Blasenreaktor ohne Wasserauskreisung im Vergleich zum Batchreaktor wird durch den Strippeffekt des IPA/Wasser-Gemisches erzielt.

Aus Tab. 1 geht außerdem der nahezu vollständige Umsatz der Myristinsäure im Blasenreaktor mit Wasserauskreisung über die Membran deutlich hervor. Zusätzlich sind die korrespondierenden Permeatflüsse angegeben.

## Tabelle 1

### Molverhältnis 2,45 : 1

| Versuchsdauer (h) | Blasenreaktor ohne Wasserauskreisung Umsatz Myristinsäure | Blasenreaktor mit Wasserauskreisung Umsatz Myristinsäure | spez. Fluß (kg/m$^2$h) |
|---|---|---|---|
| 0.0 | 0 | 0 | --- |
| 0.5 | 0,178 | 0,151 | 0,05 |
| 1.0 | 0,398 | 0,369 | 0,175 |
| 1.5 | 0,537 | 0,562 | 0,137 |
| 2.0 | 0,618 | 0,682 | 0,127 |
| 3.0 | 0,710 | 0,845 | 0,100 |
| 4.0 | 0,767 | 0,924 | 0,025 |
| 5.0 | 0,808 | 0,962 | 0,022 |
| 6.0 | 0,833 | 0,975 | 0,011 |
| 7.0 | 0,853 | 0,981 | 0,005 |
| 8.0 | 0,865 | 0,987 | 0,001 |
| 9.0 | 0,877 | | |
| 10.0 | 0,887 | | |
| 11.0 | 0,896 | | |
| 12.0 | 0,904 | | |
| 13.0 | 0,908 | | |
| 14.0 | 0,910 | | |

Gleichgewichtsumsatz Blasenreaktor ohne Wasserauskreisung: 0,910
Gleichgewichtsumsatz Batchreaktor ohne Wasserauskreisung: 0,808

$$\text{Umsatz} = 1.- \frac{\text{mol Myristinsäure}}{\text{mol}_0 \text{ Myristinsäure}}$$

In einem weiteren Versuch wurde ebenfalls bei einer spez. Membranfläche von 115,5 m$^2$/m$^3$ Myristinsäure das molare Verhältnis der Edukte reduziert (Tabelle 2). Im Gegensatz zum Reaktionsverhalten im Batchreaktor kann im Blasenreaktor die Reaktionszeit durch Reduktion des Überschusses der Nichtschlüsselkomponente im Edukt (im Beispiel IPA) minimiert werden. Dies ist dadurch zu erklären, daß vorwiegend Reaktionswasser und kaum noch IPA in die Dampfphase gelangt, dadurch der Partialdruck des Wassers im Dampf und somit die Triebkraft der Permeation erhöht wird.

Hieraus ist ersichtlich, daß die Reaktion stöchiometrisch (Molverhältnis 1 : 1) durchgeführt werden kann. Eine Aufarbeitung des Produktes, wie sie bei höheren Molverhältnissen immer anfällt, kann auf diese Weise minimiert werden, ggf. ganz entfallen.

Tabelle 2

| Versuchsdauer (h) | Umsatz Blasenreaktor mit Wasserauskreisung | Umsatz Blasenreaktor mit Wasserauskreisung |
|---|---|---|
| | Molverhältnis 2,45 : 1 | Molverhältnis 1,185:1 |
| | Umsatz Myristinsäure | Umsatz Myristinsäure |
| 0,0 | 0 | 0 |
| 0,5 | 0,151 | 0,314 |
| 1,0 | 0,369 | 0,631 |
| 1,5 | 0,562 | 0,839 |
| 2,0 | 0,682 | 0,920 |
| 3,0 | 0,845 | 0,978 |
| 4,0 | 0,924 | 0,993 |
| 5,0 | 0,962 | 0,996 |
| 6,0 | 0,975 | 0,999 |
| 7,0 | 0,981 | 1,0 |
| 8,0 | 0,987 | 1,0 |

**Bezugszeichenliste**

1 Blasenreaktor, Reaktor, Reaktionskolonne
2 Membranmodul
3 Tropfenabscheider
4 Flüssigkeitspumpe, Gasverdichter
5 Vorlagebehälter
6 Permeatkondensator
7 Permeatsammelbehälter
8 Vakuumanlage
9 Zuleitung (Dampfphase)
10 Dampfphase
11 Kondensator
12 Verdampfer
13 Leitung (Flüssigkeit)
14 Leitung (Abscheider)

**Patentansprüche**

1. Verfahren zum Durchführen einer Gleichgewichtsreaktion, bei der eines oder mehrere Produkte mittels Dämpfepermeation abgetrennt werden, wobei die Dampfphase (10) des Reaktionsgemisches im Reaktor (1) dem zur Durchführung der Dämpfepermeation vorgesehenen Membranmodul (2) als Feed zugeleitet wird,
dadurch gekennzeichnet,
daß die Reaktion in einem Blasenreaktor (1) durchgeführt und das leichtersiedende Edukt durch das auf Reaktionstemperatur gehaltene, flüssige und getrennt vorgelegte Edukt geleitet wird, wobei die Reaktionstemperatur und der Reaktordruck so eingestellt werden, daß bei Reaktionstemperatur das leichtersiedende Edukt und das abzutrennende Produkt dampfförmig vorliegen, daß die Dämpfepermeation außerhalb des Reaktors (1) unter geeigneter Temperatur, die abhängig vom Membranmaterial ist, vorgenommen und das bei der Dämpfepermeation entstehende Retentat zurückgeleitet und gemeinsam mit dem leichtersiedenden Edukt in den Reaktor (1) geleitet wird.

2. Verfahren nach Anspruch 1 zum Durchführen einer Veresterungsreaktion, bei dem Wasserdampf mittels Dämpfepermeation abgetrennt wird.

3. Verfahren nach Anspruch 1 oder 2,
dadurch gekennzeichnet,

EP 0 535 073 B1

daß man das Membranmodul (2) und/oder seine vom Reaktor (1) herführende Zuleitung (9) entsprechend der gewünschten Membrantemperatur erwärmt bzw. kühlt.

**4.** Verfahren nach einem der Ansprüche 1 bis 3,
dadurch gekennzeichnet,
daß das Retentat zusammen mit dem leichtersiedenden Edukt in flüssigem Zustand in den Reaktor (1) geleitet wird.

**5.** Verfahren nach einem der Ansprüche 1 bis 3,
dadurch gekennzeichnet,
daß das Retentat zusammen mit dem leichtersiedenden Edukt in gasförmigem Zustand in den Reaktor (1) geleitet wird.

**6.** Verfahren nach Anspruch 1 oder 5,
dadurch gekennzeichnet,
daß das höhersiedende flüssige und das leichtersiedende dampfförmige Edukt im Gegenstrom durch eine Rührkesselkaskade geleitet werden und die gasförmige Phase des Reaktionsgemisches erst nach Durchlaufen der Kaskade dem Membranmodul (2) zugeleitet wird.

**7.** Verfahren nach einem der Ansprüche 1 bis 5,
dadurch gekennzeichnet,
daß die Edukte durch eine Gegenstromkolonne geleitet werden und das entstehende gasförmige Reaktionsprodukt dem Membranmodul (2) zugeleitet wird.

**8.** Verfahren nach einem der Ansprüche 1 bis 7,
dadurch gekennzeichnet,
daß den Brüden vor der Zuleitung zum Membranmodul (2) eine Vorrichtung zur Abscheidung mitgerissener Flüssigkeit vorgeschaltet ist.

## Claims

**1.** A process for conducting an equilibrium reaction in which one or more products is/are separated off by vapour permeation, the vapour phase (10) of the reaction mixture in the reactor (1) being delivered as feed to the membrane module (2) in which the vapour permeation is to be carried out, characterized in that the reaction is carried out in a bubble reactor (1) and the lower-boiling educt is passed through the liquid educt separately introduced which is kept at the reaction temperature, the reaction temperature and the reactor pressure being adjusted so that the low-boiling educt and the product to be removed are present in vapour form at the reaction temperature; in that the vapour permeation is carried out outside the reactor (1) at a suitable temperature which is dependent on the membrane material and the retentate formed during the vapour permeation is returned to the reactor (1) together with the lower-boiling educt.

**2.** A process as claimed in claim 1 for carrying out an esterification reaction in which steam is removed by vapour permeation.

**3.** A process as claimed in claim 1 or 2, characterized in that the membrane module (2) and/or its feed pipe (9) coming from the reactor (1) is/are optionally heated or cooled in dependence upon the required membrane temperature.

**4.** A process as claimed in any of claims 1 to 3, characterized in that the retentate is introduced into the reactor (1) in liquid form together with the lower-boiling educt.

**5.** A process as claimed in any of claims 1 to 3, characterized in that the retentate is introduced into the reactor (1) in gaseous form together with the lower-boiling educt.

**6.** A process as claimed in claim 1 or 5, characterized in that the higher-boiling liquid educt and the lower-boiling vaporous educt are passed in countercurrent through a cascade of stirred tanks and the gaseous phase of the reaction mixture is only delivered to the membrane module (2) after passing

9

through the cascade.

7. A process as claimed in any of claims 1 to 5, characterized in that the educts are passed through a countercurrent column and the gaseous reaction product formed is delivered to the membrane module (2).

8. A process as claimed in any of claims 1 to 5, characterized in that the vapours are preceded by a unit for removing entrained liquid before the feed pipe to the membrane module (2).

**Revendications**

1. Procédé pour réaliser une réaction d'équilibre, au cours de laquelle un ou plusieurs produits sont séparés au moyen de la perméation de vapeur, dans lequel la phase vapeur (10) du mélange réactionnel dans le réacteur (1) est amenée comme alimentation au module de membrane (2) prévu pour réaliser la perméation de vapeur,
caractérisé en ce que la réaction est réalisée dans un réacteur à bulles (1) et l'éduct à point d'ébullition plus bas est amené liquide par l'éduct produit séparément maintenu à la température de réaction, tandis que la température de réaction et la pression du réacteur sont ainsi ajustées, de façon qu'à la température de réaction l'éduct à plus bas point d'ébullition et le produit à séparer se présentent sous la forme de vapeur, que la perméation de vapeur soit entreprise en dehors du réacteur à une température appropriée, qui est fonction du matériau de la membrane et que le rétentat se formant lors de la perméation de la vapeur soit reconduit et soit amené en commun avec l'éduct à point d'ébullition plus bas dans le réacteur (1).

2. Procédé selon la revendication 1 pour réaliser une réaction d'estérification dans laquelle la vapeur d'eau est séparée au moyen d'une perméation de vapeur.

3. Procédé selon les revendications 1 ou 2, caractérisé en ce que,
on chauffe ou refroidi le module de membrane (2) et/ou sa conduite (9) provenant du réacteur (1) en conformité à la température souhaitée de la membrane.

4. Procédé selon une des revendications 1 à 3, caractérisé en ce que,
le rétentat en commun avec l'éduct à point d'ébullition plus bas est amené à l'état liquide dans le réacteur (1).

5. Procédé selon une des revendications 1 à 3, caractérisé en ce que,
le rétentat en commun avec l'éduct à point d'ébullition plus bas est amené sous forme gazeuse dans le réacteur (1).

6. Procédé selon les revendications 1 ou 5, caractérisé en ce que,
l'éduct liquide à point d'ébullition plus élevé et l'éduct sous forme de vapeur à point d'ébullition plus bas sont amenés à contre courant à travers une cascade de caissons d'agitation et la phase gazeuse du mélange réactionnel est amenée juste après le passage de la cascade au module de membrane (2).

7. Procédé selon une des revendications 1 à 5, caractérisé en ce que,
les éducts sont guidés à travers une colonne à contre courant et le produit réactionnel se formant sous forme gazeuse est amené au module de membrane (2).

8. Procédé selon une des revendications 1 à 7, caractérisé en ce que,
un dispositif de séparation de liquide entraîné est monté en amont des vapeurs avant la conduite d'amenée au module de membrane (2).

Fig . 1

Fig . 2

Fig . 3